# EUROPEAN PATENT SPECIFICATION

(11) **EP 2 731 512 B1**
(45) Date of publication and mention of the grant of the patent: **07.10.2020**
(21) Application number: 12740926.6
(22) Date of filing: 12.07.2012
(51) Int. Cl.: A61B 17/17, A61F 2/46

(54) **METHOD OF MANUFACTURING A SURGICAL INSTRUMENT FOR THE POSITIONING OF AN ALIGNMENT ELEMENT**
VERFAHREN ZUR HERSTELLUNG EINES CHIRURGISCHEN INSTRUMENTS ZUR POSITIONIERUNG EINES AUSRICHTUNGSELEMENTS
PROCÉDÉ DE FABRICATION D'UN INSTRUMENT CHIRURGICAL SERVANT À POSITIONNER UN ÉLÉMENT D'ALIGNEMENT

(30) Priority: 12.07.2011 EP 11173606; 12.07.2011 US 201113180688
(43) Date of publication of application: 21.05.2014
(60) Divisional application: 20187534.1
(73) Proprietor: Materialise N.V., 3001 Leuven (BE)
(72) Inventor: HANANOUCHI, Takehito, Hyogo 661-0967 (JP); VANGENEUGDEN, Dieter, 3900 Overpelt (BE)
(74) Representative: EIP
(86) International application number: PCT/EP2012/063676
(87) International publication number: WO 2013/007785

(56) References cited:
- WO-A1-2010/124164
- WO-A1-2011/029911
- WO-A1-2011/060536
- US-A- 5 976 149
- US-A1- 2004 199 258
- US-A1- 2006 161 167
- US-A1- 2008 262 499
- US-A1- 2010 016 984
- US-A1- 2010 082 035
- US-A1- 2010 087 829
- US-A1- 2011 093 086

## Description

### FIELD OF THE INVENTION

The present invention relates to methods of manufacturing surgical instruments for positioning an alignment element based on pre-operational planning.

### BACKGROUND

In most joint arthroplasty, replacement and/or reconstruction surgery procedures, and in particular in hip and shoulder joint surgery, a joint is replaced by a prosthetic implant. The main goal of such interventions is to relieve (arthritic) pain and/or to restore severe physical joint damage. When prosthesis fails, a revision surgery is carried out. However, this procedure is technically more difficult and time-consuming than the primary intervention and the outcome is often less satisfactory, both because there is less bone stock to work with and because the removal of adherent cement or prosthetic components may result in fracture or perforation of the bone. Furthermore, with each successive joint revision, the risk of infection and symptomatic loosening of the prosthesis may increase substantially. Accordingly, one of the most important aspects of joint surgery procedures is the correct, accurate and stable placement of the primary implant.

Correct implant placement is important in shoulder surgery, and particularly important in hip surgery. The majority of acetabular implants used in hip surgery are currently placed using the press-fit technique. In this technique, the patient's acetabulum is first reamed with a sequence of hemispherical reamers with increasing diameters, such that a hemispherical cavity is created at the location where the implant should be placed. The final (largest) reamer typically has a diameter smaller than that of the implant. In a further step, the implant is attached to an impactor and placed upon the pelvis of the patient, such that the implant supports on the rim of the reamed cavity and the orientation of the implant is anatomically suitable. Finally, the impactor is hit with a hammer until the implant sits inside the reamed cavity. Thereafter, the implant is released from the impactor.

The general consensus in the field is that the orientation of the implant determines the success of the surgery and the lifespan of the implant (Hayakawa et al., Archives of orthopedic and trauma surgery Vol. 129 (2009):1151-1156). However, the current procedure shows several shortcomings for obtaining a good orientation. Indeed, the only anatomical visual reference during final placement is the orientation of the transverse ligament (Pearse et al., Hip international Vol. 18 (2008):7-10), to which the top plane of the implant should be oriented in parallel. Accordingly, rotation around the axis of the transverse ligament remains a variable parameter. In addition, the transverse ligament is generally obscured from the surgeon's view, further hampering the orientation process. Furthermore, the impactor and hammer are both rather bulky, making it difficult to keep the impactor in a stable orientation.

Few practical solutions have been proposed for these problems. US patent application 2009/0163922 (Meridew, Metzger) describes a patient-specific guide to be positioned and optionally attached to the acetabular rim, designed to interface with the impactor so as to enforce the correct orientation. However, it is very doubtful whether such a device could be able to withstand the momentum applied to it during impaction.

US patent application 2010/0082035 and International patent application WO 2011/060536 disclose patient-specific surgical instruments for facilitating implantation of an acetabular cup prosthesis in a bone of a patient. The guides may be used for positioning a guide pin bone guide pin to the patient's acetabulum. However, such guides only provide a limited accuracy.

International patent publication number WO 2010/124164 describes a method and apparatus for positioning an acetabular cup in a desired alignment in relation to patient's hemipelvis.

US patent publication number US 2010/087829 describes a patient-specific guiding system for guiding an instrument relative to a portion of an anatomical feature of a patient.

International patent publication number WO 2011/029911 describes an adaptable therapeutic, diagnostic or surgical guide for an intra-operative adjustment of a guidance element to a pre-planned position.

US patent publication number US 2008/262499 describes a spring-loaded, self-retaining, clamp like alignment device used to guide the placement of an acetabular component during total hip arthroplasty.

US patent publication number US 2004/199258 describes a modular acetabular cup for surgical treatment for hip join reconstruction in cases of total recovery of hip implant, and an anchoring screw for fixing a prosthetic implant such as said acetabular cup.

Accordingly, there is a need for alternative and improved surgical devices, and in particular surgical guiding instruments, which provide the ability to correctly and accurately insert, place and orient an implant into a patient's joint.

### SUMMARY OF THE INVENTION

The present disclosure relates to methods for manufacturing surgical instruments for use in arthroplasty. The instruments are intended for facilitating surgery on ball-and-socket joints in the human or animal body. In surgery on the human body, the instruments are therefore useful for hip and shoulder joint surgery, particularly for the positioning of an acetabular cup implant or glenoid implant. The surgical instruments allow for positioning of an alignment element, where the desired position and orientation of the alignment element is typically based on pre-operational planning. The alignment element can be an indicator pin, wire, screw or drill, which acts as a navigator for the surgeon to address an optimal pre-operationally planned implant alignment direction, in the reaming and/or the impacting phase of the surgical procedure.

The surgical instruments manufactured according to the present disclosure are surgical fixtures. In a first aspect, the present invention provides methods for manufacturing surgical fixtures for positioning an alignment element. The fixtures comprise one or more patient-specific contact elements which together fit onto areas on a socket of a ball-and- socket joint, onto areas around said socket and/or onto the rim of said socket in at least three contact points. Where the socket is a glenoid cavity, the areas around the socket may include the glenoidal rim and the periglenoidal region (e.g. infraglenoidal tuberculum, supraglenoidal tuberculum and collum scapulae, etc.) and might include the acromion and processus coracoideus (coracoid process). Where the socket is an acetabulum, the areas around the socket may include the periacetabular region (e.g. the limbus acetabuli, sulcus supra-acetabularis, superior ramus, etc.).

In particular embodiments, the contact points have an arrangement wherein the angle between the line connecting one contact point and the center of the circle or ellipse best fitting the socket rim and the line connecting the adjacent contact point and said center is never greater than 180°. The surgical fixtures further comprise a positioning element which is rigidly attached to the fixture. This positioning element is provided with one or more holes which allow the insertion of the alignment element. Additionally, in particular embodiments, the positioning element is detachable from the rest of the fixture.

In particular embodiments, one (of the) contact element(s) is positioned on the fixture such that, when positioned on the bone, it interacts with an anatomical feature present on the rim of the socket or on the bone in or around the socket. In further embodiments, this anatomical feature is the posterior notch of the transverse ligament, or the coracoid process.

The invention provides methods for manufacturing patient-specific surgical fixtures for positioning an alignment element on the acetabular socket of a pelvic bone as defined in claim 1, comprising a one-piece structure with one or more patient-specific contact elements which fit onto one or more discrete areas on the socket. More particularly these one or more contact elements fit around and/or onto the acetabular rim, whereby at least one of said one or more contact elements interact with the posterior notch of the transverse ligament of the rim of said acetabulum so as to ensure a tight fit of said fixture on the acetabular rim. The fixture further comprises a positioning element which can be or is rigidly attached to said one-piece structure comprising contact elements, which positioning element is provided with one or more holes which allows the insertion of said alignment element.

In certain embodiments of the fixtures described herein, the surgical fixtures comprise at least two contact elements, or at least three contact elements. Together, the contact elements fit onto areas on the socket, around the socket and/or on the socket rim in at least three contact points, whereby the contact points have an arrangement wherein the angle between a line drawn between one contact point and the center of the circle or ellipse best fitting the socket rim and a line drawn between the adjacent contact point and said center is never greater than 180°. In further embodiments, the surgical fixture comprises at least two contact elements, wherein the positioning element corresponds to one of the contact elements.

In certain embodiments of the fixtures described herein, the one or more holes in the positioning element allow the insertion of said alignment element outside of said socket.

In certain embodiments of the fixtures described herein, the positioning element is detachable from the rest of said fixture.

In certain embodiments of the fixtures described herein, the one or more contact elements which fit onto areas on the rim of said socket in at least three contact points are irreversibly fixed to each other.

As indicated hereabove, the positioning element may be detachable from the rest of the fixture. In particular embodiments, the connection between the positioning element and the rest of the fixture is adapted or weakened, such that the positioning element can be detached from the rest of the fixture by breaking said connection with surgical cutting elements. In other embodiments, the connection between the positioning element and the rest of the fixture is ensured by an element selected from a dovetail coupling, interlocking features, a pinned system and a snap-fit mechanism.

In particular embodiments, the surgical fixtures further comprise a connecting structure, wherein the positioning element and/or one or more of the one or more contact elements extend from the connecting structure.

As indicated hereabove, the positioning element comprises one or more holes. In certain embodiments, the position and/or direction of at least one hole is in accordance with pre-operational planning. In certain embodiments, at least one hole is part of a drill guide. In certain embodiments, the positioning element comprises a first and a second hole with a different diameter, wherein the first hole allows the insertion of the alignment element and wherein the second hole allows the insertion of a fixation element. In further embodiments, the positioning element comprises two or more holes which allow the insertion of a fixation element. In particular embodiments, the alignment element is selected from the group comprising a pin, a wire, a screw and a drill.

In particular embodiments, the surgical fixtures manufactured according to the present invention are manufactured via additive manufacturing.

The present invention provides methods for the manufacture of the patient-specific surgical fixtures according to the embodiments described above. The methods comprise the steps of:
i. obtaining volume information of the socket of a ball-and-socket joint from a patient;
ii. obtaining the installation direction of a socket implant for the patient;
iii. identifying and selecting parts of the bone surrounding the implant zone which are suitable for inserting an alignment element;
iv. identifying and selecting parts of the bone in or surrounding the implant zone which are suitable for use as a base for the contact surface or surfaces of the surgical fixture;
v. designing and producing a surgical fixture based on the information obtained in steps i, ii, iii, iv and v.

The patient may be an animal or human patient. Therefore the socket may be any socket of a ball-and-socket joint in an animal or human body. In human patients, the socket of a ball-and-socket joint may be an acetabulum or a glenoid cavity. In embodiments of the invention, the socket is an acetabulum. In comparative embodiments, the socket is a glenoid cavity.

The surgical fixtures manufactured according to the present invention allow for a fast and accurate positioning of an indicator pin, wire, screw or drill, and allow for an efficient removal of the fixture from the anatomy after use.

Preferred embodiments are according to the dependent claims.

### BRIEF DESCRIPTION OF THE DRAWINGS

The following figures of specific embodiments of the invention are merely exemplary in nature and are not intended to limit the present teachings, their application or uses. Throughout the drawings, corresponding reference numerals indicate like or corresponding parts and features.

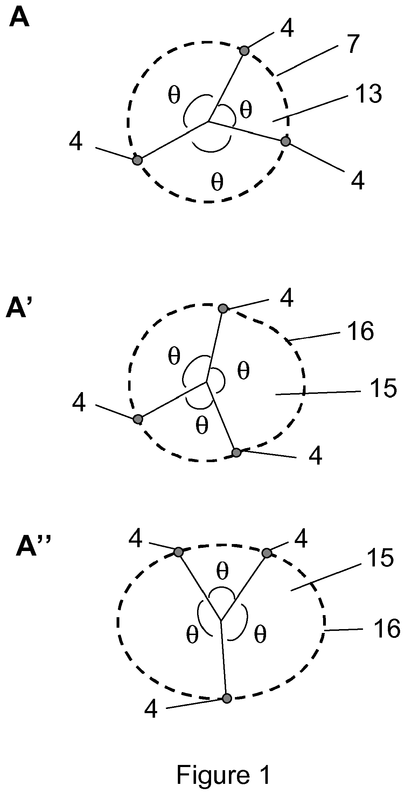

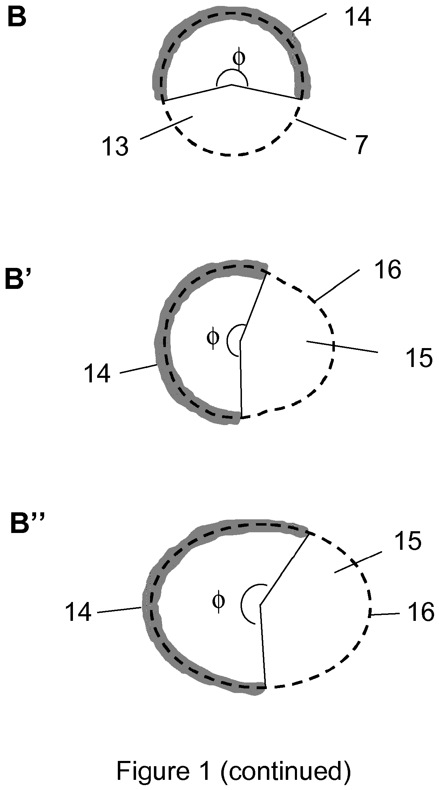

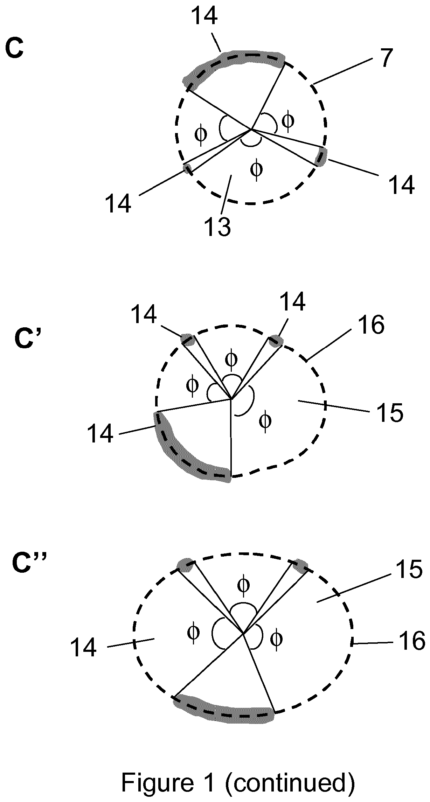

**Figure 1**A, A', A": Schematic representation of the relative position of patient-specific contact points (4) and their orientation relative to the acetabular rim (7) or glenoid cavity rim (16) and the acetabulum (13) or glenoid cavity (15) according to a particular embodiment. B, B', B", C, C', C": Schematic representation of the arrangement and shape of patient-specific contact surfaces (14) and their orientation relative to the acetabular rim (7) or glenoid cavity rim (16) and the acetabulum (13) or glenoid cavity (15) according to a particular embodiment.

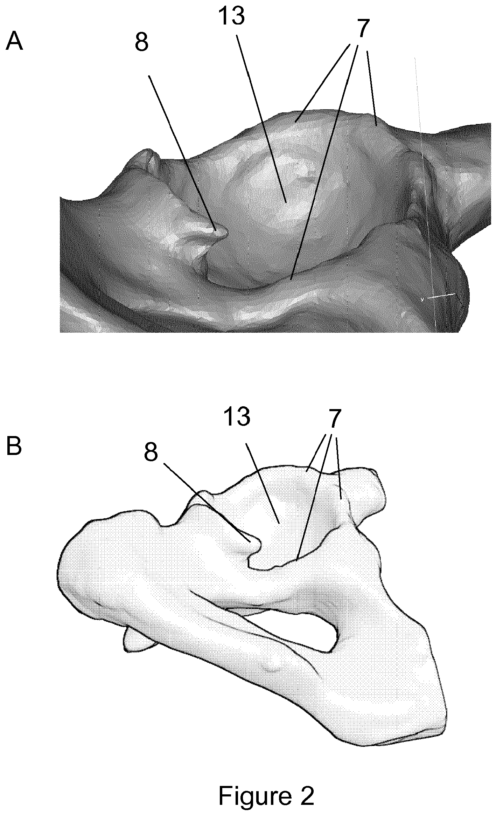

**Figure 2**A, B: Drawing of an acetabulum (13) surrounded by the acetabular rim (7) and the posterior notch of the transverse ligament (8).

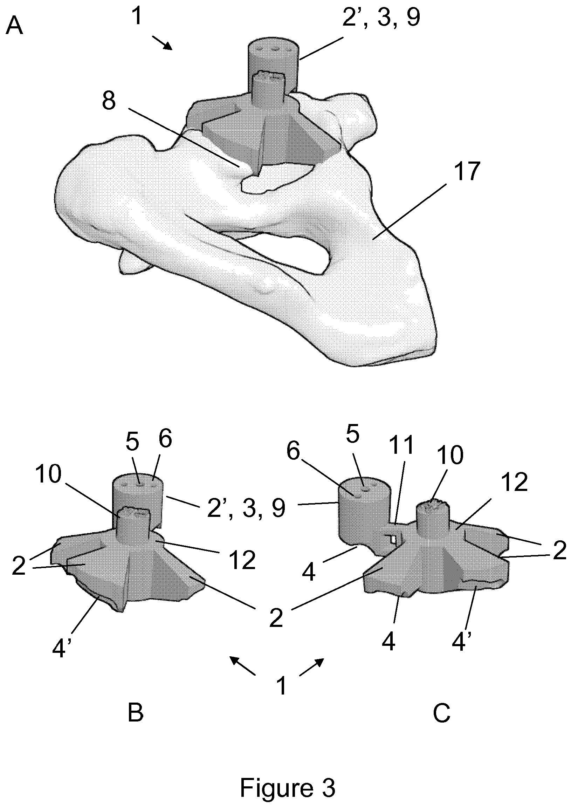

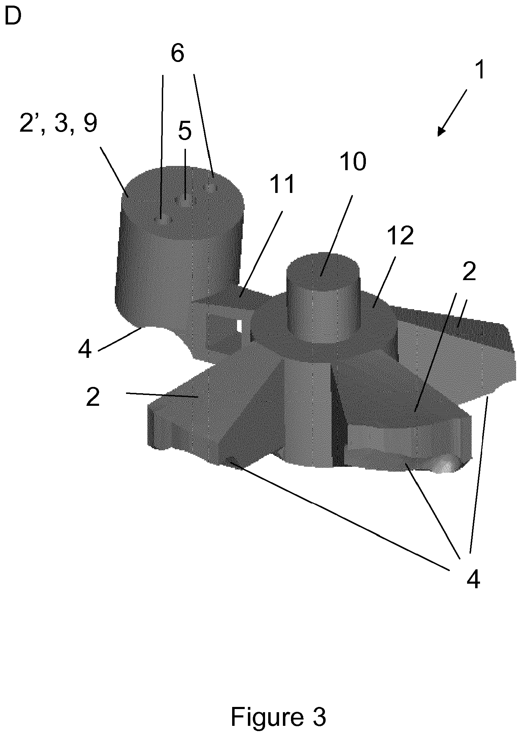

**Figure 3** Surgical fixture (1) which may be manufactured according to a particular embodiment of the present invention, positioned onto an acetabulum (A), and not positioned on a bone (B, C). The fixture comprises a central connecting structure (12), a positioning element (3) and four contact elements (2, 2'), each comprising a contact surface (4). The fixture also comprises an extension (10) for manipulation of the fixture. The positioning element (3) also functions as a drill guide (9), comprises holes (5, 6) and is connected to the rest of the fixture via a weakened connection (11).

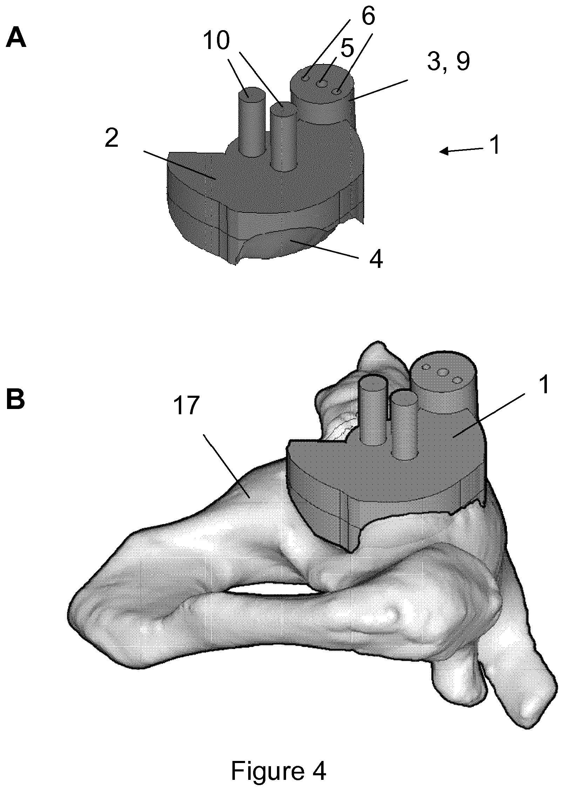

**Figure 4** A: Surgical fixture (1) which may be manufactured according to a particular embodiment of the present invention, comprising a positioning element (3) and a contact element (2) comprising a contact surface (4). The fixture further comprises two extensions (10). The positioning element (3) is also a drill guide (9), comprises holes (5, 6). B: the same surgical fixture (1) positioned on an acetabulum of a pelvic bone (17).

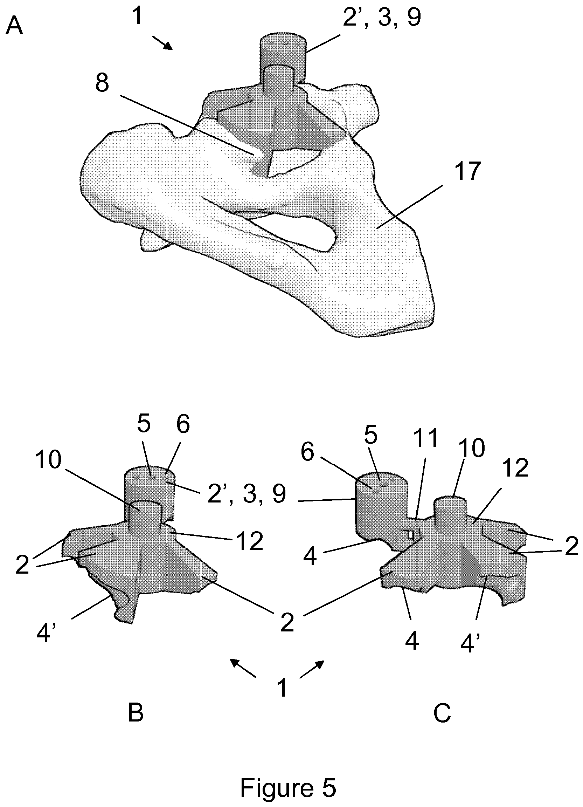

**Figure 5** A: surgical fixture (1) which may be manufactured according to a particular embodiment of the present invention, positioned on an acetabulum (7) of a pelvic bone. B, C: surgical fixture (1) which may be manufactured according to a particular embodiment of the present invention, not positioned on a bone

In the figures, the following numbering is used:
1 - surgical fixture; 2, 2' - contact element; 3 - positioning element; 4, 4' - contact point; 5 - hole for insertion of alignment element; 6 - hole for fixation element; 7 - acetabular rim; 8 - posterior notch of the transverse ligament; 9 - drill guide; 10 - extension; 11 - adapted connection; 12 - connecting structure; 13 - acetabulum; 14 - contact surface; 15 - glenoid cavity; 16 - glenoid cavity rim; 17 - pelvic bone

### DETAILED DESCRIPTION

The present invention will be described with respect to particular embodiments but the invention is not limited thereto but only by the claims. Any reference signs in the claims shall not be construed as limiting the scope thereof.

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms "comprising", "comprises" and "comprised of' also include the term "consisting of'.

Furthermore, the terms first, second, third and the like in the description and in the claims, are used for distinguishing between similar elements and not necessarily for describing a sequential or chronological order, unless specified. It is to be understood that the terms so used are interchangeable under appropriate circumstances and that the embodiments of the invention described herein are capable of operation in other sequences than described or illustrated herein.

The term "about" as used herein when referring to a measurable value such as a parameter, an amount, a temporal duration, and the like, is meant to encompass variations of +/-10% or less, preferably +/-5% or less, more preferably +/-1% or less, and still more preferably +/-0.1 % or less of and from the specified value, insofar such variations are appropriate to perform in the disclosed invention. It is to be understood that the value to which the modifier "about" refers is itself also specifically, and preferably, disclosed.

The recitation of numerical ranges by endpoints includes all numbers and fractions subsumed within the respective ranges, as well as the recited endpoints.

Unless otherwise defined, all terms used in disclosing the invention, including technical and scientific terms, have the meaning as commonly understood by one of ordinary skill in the art to which this invention belongs. By means of further guidance, definitions for the terms used in the description are included to better appreciate the teaching of the present invention. The terms or definitions used herein are provided solely to aid in the understanding of the invention.

The present invention provides methods for manufacturing a patient-specific surgical fixture for positioning an alignment element. The alignment element may be used for indicating a (pre- operationally planned) direction and/or position for an implant. The fixture comprises one or more patient specific contact elements, which fit onto areas on or around a socket, and/or on the socket rim; the fixture further comprises a positioning element for positioning the alignment element.

The present invention relates to the field of implant surgery, more particularly implants which are placed into a socket of a ball-and-socket joint. For human patients, this is an acetabular cup implant and/or a glenoid implant. The term "acetabular cup implant" as used herein refers to the component of a prosthetic hip implant which is placed into the acetabulum of a patient. The acetabulum is a concave surface of the pelvis, where the head of the femur meets with the pelvis, thus forming the hip joint. The term "glenoid implant" as used herein refers to a component of a prosthetic shoulder implant which is placed into or onto the glenoid cavity of a patient. Such implants may be used in a (total) shoulder arthroplasty or reverse (total) shoulder arthroplasty. The glenoid cavity, also known as glenoid fossa (of the scapula), is a shallow surface, which is located on the lateral angle of the scapula. This cavity forms the glenohumeral joint along with the humerus.

The terms "rim" and "socket rim" as used herein refer to the edge of a socket. Usually, this is a substantially convex edge of the concave bone surface which forms the socket. For human patients, particular examples are the acetabular rim and/or the glenoid rim. The term "acetabular rim" as used herein refers to the edge of the acetabulum, more particularly the substantially convex edge of the concave surface of the pelvis which forms the acetabulum. The term "glenoid rim" as used herein refers to the edge of the glenoid cavity, more particularly the substantially convex edge of the concave surface of the scapula which forms the glenoid cavity.

The term "socket" as used herein in the context of a ball joint, refers to a socket of a ball-and-socket joint of the human or animal body. For human patients, typical examples include the acetabulum and/or the glenoid cavity.

The term "alignment element" as used herein refers to an element which facilitates the correct positioning of an implant into or onto an anatomical socket, for example by indicating a certain location and/or direction for positioning and/or by physically guiding the implant or an implant guide to a certain location. Without such an element, the implant may be positioned incorrectly, leading to suboptimal functioning of the prosthesis and discomfort to the patient.

The terms "surgical fixtures" and "fixture" as used herein refer to (patient-specific) surgical tools that can be positioned onto an anatomical part of a patient and that help a surgeon in the positioning of an alignment element.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In a first aspect, the present invention provides methods for manufacturing surgical instruments for facilitating the positioning of an implant into or onto a socket of a ball-and-socket joint in the body of an animal or human patient. More particularly, in the context of humans, the present invention provides methods for manufacturing surgical fixtures for positioning an alignment element, which can be used for positioning an acetabular cup implant or a glenoid implant. The surgical fixtures are however equally useful for use in animals.

The surgical fixtures which may be manufactured according to the present invention comprise at least one patient-specific contact element, i.e. a part of the surgical fixture which is used to ensure the correct positioning of the surgical fixture by contacting specific locations on the patient's anatomy.

The (one or more) patient-specific contact elements allow the surgeon to obtain the correct position of the surgical fixture onto the socket and/or socket rim, according to pre-operational planning. Indeed, the one or more contact element(s) (together) fit onto specific areas on or around the socket and/or socket rim (with or without cartilage or other soft tissue). In particular embodiments, the one or more contact elements fit onto the rim of the socket in at least three contact points. More particularly, if the fixture comprises only one contact element, that contact element fits onto specific areas on or around the socket and/or socket rim in at least three contact points. Alternatively, if the fixture comprises two or more contact elements, the contact elements are arranged such that together, they fit onto specific areas on or around the socket and/or socket rim in at least three contact points. The contact points have an arrangement, preferably so as to surround the socket, whereby the angle between:
- the line connecting one contact point and the center of the circle or ellipse best fitting the rim of the socket, and
- the line connecting the adjacent contact point and the center of the circle or ellipse best fitting the rim of the socket,
is never greater than 180°.
In particular embodiments, the angle between the contact points (as determined by lines connecting each contact point with the center of the circle of ellipse) is never greater than 175, 170, 165, 160, 155, 150, 145, 140, 135, 130, 125, 120, 115, 110, 105, 100,95 or 90°. This will be explained in further detail herein below.

In particular embodiments, the contact elements which extend over these three contact points are irreversibly attached to each other. In particular embodiments, at the least the part of the fixture comprising these contact elements is made as a one piece structure. This implies that the contact elements are fitted onto the rim of the socket simultaneously.

The surgical fixtures which may be manufactured according to the present invention further comprise a positioning element. The positioning element is a part of the fixture which is used for placing the alignment element onto or into the bone in or surrounding the socket in a pre-operationally planned position. In particular embodiments, the positioning element is placed such that it guides the alignment element in the bone surrounding the socket. The bone surrounding the socket suitable for placing the alignment element is, for example, the bone in the periacetabular region (e.g. the limbus acetabuli, sulcus supra- acetabularis, superior ramus, etc.) or periglenoidal region (e.g. infraglenoidal tuberculum, supraglenoidal tuberculum and collum scapulae, etc.). Therefore, the positioning element is provided with at least one hole or slit which either guides or allows the insertion of an alignment element. The positioning element is either integrated in or rigidly attached to the rest of the fixture comprising the contact elements. In particular embodiments, the positioning element is a structure rigidly attached to the contact elements. In these embodiments, the contact element(s) and the positioning element in the surgical fixture, allow for the correct positioning of the fixture on the bone and thus the correct positioning of an alignment element. Indeed, the fixed position of the positioning element relative to the contact element(s) and the rigidity of the fixture ensure that once secured in the right position on the bone via the contact element(s), the positioning element allows the accurate positioning of an alignment element, according to pre-operational planning.

The positioning element is attached rigidly to the fixture. Thus the connection between the positioning element and the rest of the fixture is not flexible or resilient. This means that the relative position of the positioning element and the contact element(s) of the fixture is fixed, as long as the positioning element is attached to the fixture. Accordingly, the position and orientation of the positioning element on the socket is determined by the position of the contact elements on the bone. Moreover, this ensures that the relative position of the positioning element and the contact element(s) remains fixed during positioning the fixture on the socket joint, and therefore ensures an accurate positioning of the alignment element via the positioning element, according to pre- operational planning.

In particular embodiments, the fixtures are characterized by the fact that the positioning element of the fixture is detachable, i.e. detachable from the rest of the surgical fixture. This can be ensured by the fact that the positioning element is attached to the surgical fixture by a rigid connection which is weakened and/or adapted such that it can be broken when applying force, or cut or removed using surgical instruments. Additionally or alternatively, the positioning element may be removably attached to the rest of the surgical fixture via features which allow a rigid, reversible connection of the positioning element, e.g. interlocking features, a dovetail coupling, a pinned system, a snap-fit system and the like. The fact that the positioning element is detachable facilitates the removal of the surgical fixture from the bone after the alignment element is introduced into the bone through the positioning element.

The desired position of the alignment element for guiding the placement of an implant is determined by pre-operative planning. Moreover, using preoperative planning optimal contact points for the contact element(s) of the surgical fixture which may be manufactured according to the present invention can be determined. Indeed, while the number and shape of the contact elements may vary, the contact points comprised therein determine the stability of the fixture. The optimal position of the contact points ensures positional stability of the surgical fixture onto the socket. Optimally, to ensure positional stability of the fixture onto an acetabulum, the contact points have a circular or substantially circular arrangement, preferably so as to surround the acetabulum (13), wherein the angle e defined by two adjacent contact points is never greater than 180°. In other words, the angle between the line connecting one contact point (4) and the center of the circle best fitting the acetabular rim (7) and the line connecting the adjacent contact point (4) and said center is never greater than 180°. This is shown in Figure 1A. Hereby the acetabulum is considered to have a substantially circular shape.

The glenoid cavity on the other hand, can be considered piriform. Thus, the glenoid cavity comprises a substantially circular shape on one side, but tapering towards the other side. Therefore, to ensure positional stability of the fixture onto a glenoid cavity (15), the contact points have a circular or substantially circular arrangement around the circumference of the rim of the glenoid cavity. More particularly, the angle θ formed by a first line drawn between one contact point (4) and the center of the circle best fitting the (substantially circular part of) the glenoid cavity rim (16) and a second line drawn between the adjacent contact point (4) and said center is never greater than 180°. This can also be expressed in terms of the sector angle defined by two adjacent contact points (4) which is never greater than 180°. This is shown in Figure 1 A'.

Alternatively, the glenoid cavity may be considered as having a roughly elliptical shape. In that case, to ensure positional stability of the fixture onto a glenoid cavity (15), the contact points have an arrangement, wherein the angle θ between the (straight) line connecting one contact point (4) and the center of the ellipse best fitting the glenoid cavity rim (16) and the (straight) line connecting an adjacent contact point (4) and said center is never greater than 180°. Again this can be expressed by the fact that the sector angle defined by two adjacent contact points is never greater than 180°. This is shown in Figure 1 A".

Thus, more generally for an undefined socket, in particular embodiments of the fixtures, the contact elements fit onto the socket and/or socket rim by contacting said socket and/or rim at different contact points. Together, the contact elements fit onto areas of (or around) a socket and/or a socket rim in at least three contact points, wherein the contact points have an arrangement, preferably so as to surround the socket, wherein the angle between a line drawn between one contact point and the center of the circle or ellipse best fitting the socket rim and a second line drawn between an adjacent contact point and said center is never greater than 180° or similarly the sector angle defined by two adjacent contact points is never greater than 180°. These contact points may all be located on the same contact element (i.e. one contact element comprises these three contact points), or distributed over two or more contact elements.

In certain embodiments, the one or more contact elements of the surgical fixtures contain, on the surface which is intended for placement on the bone (with or without cartilage or other soft tissue), patient-specific surfaces, i.e. anatomy engagement surfaces which at least partially match the surface of (or around) the socket and/or the socket rim. This implies that the contact element comprises of a plurality of contact points (corresponding to a contact surface). The positional stability of the surgical fixture onto the acetabulum is then at least in part determined by the size and position of the contact surfaces on the contact element(s). The patient specific surface of a contact element typically spans an area which varies between one square micrometer (µm²) and fifty square centimeters (cm²).

In particular embodiments, the surgical fixtures comprise only one contact element, which has a single contact surface. In particular embodiments, this contact surface (14) spans the surface of the socket (13) and/or socket rim (7) over an angle φ, of at least 180°, as schematically drawn for a circular socket (acetabulum) in Figure 1B. A piriform or elliptical socket (glenoid cavity) imposes a similar requirement (Figures 1 B' and B").

Alternatively, the surgical fixtures may comprise more than one contact element comprising a patient-specific contact surface. A stable positioning of the surgical fixture can then optimally be obtained if the contact surfaces of the different contact elements have an arrangement, on or around the socket (13) and/or socket rim (7), wherein for every pair of adjacent contact points not belonging to the same contact surface (14), the angle φ between the line drawn between one contact point and the center of the circle best fitting the socket rim (7) and a second line drawn between the adjacent contact point and said center is never greater than 180°, as schematically drawn for a circular socket (acetabulum) in Figure 1C. Again, a piriform or elliptical socket (glenoid cavity) imposes a similar requirement (Figure 1 C' and C"). Also fixtures comprising combinations of one or more contact points with one or more contact surfaces are envisaged.

Alternative or specific combinations of the above are also envisaged. In certain embodiments, as indicated above, the surgical fixtures comprise only one contact element, which comprises several patient-specific contact points and/or contact surfaces. In other embodiments, the surgical fixtures comprise at least two contact elements, each comprising one or more patient-specific contact points and/or surfaces as described hereabove. In certain embodiments, the surgical fixtures comprise three contact elements, each comprising one or more patient-specific contact points and/or surfaces as described hereabove. In further embodiments, the surgical fixtures comprise four contact elements, each comprising one or more patient-specific contact points and/or surfaces as described hereabove. In yet further embodiments, the surgical fixtures comprise (at least) five, six, seven, eight, nine, ten or more contact elements, each comprising one or more patient-specific contact points and/or surfaces as described hereabove.

It will be understood to the skilled person that, in the fixtures as described herein the different contact elements of the surgical fixtures need not make contact with the bone (with or without cartilage or other soft tissue) in the same way and need not contact the bone over their entire surface. Thus, in particular embodiments, at least one contact element contacts, at least partially, the socket rim via its contact point(s) and/or surface(s). In further embodiments, all contact elements contact, at least partially, the socket rim via the contact point(s) and/or surface(s) comprised therein. Specifically, in particular embodiments, the position and/or orientation of at least one contact element on the surgical fixture is patient-specific.

Advantageously, at least one contact element is positioned on the fixture such that its patient-specific surface corresponds to the surface of the corresponding socket in the location of a conspicuous anatomical feature in or around the socket. For the acetabulum, this is for example the posterior notch of the transverse ligament, hereinafter also referred to as "posterior notch". The posterior notch of the transverse ligament (8) of the acetabulum (13) is drawn in Figure 2A and B. For the glenoid cavity, this is for example the coracoid process (processus coracoideus); this is a small hook- like structure on the lateral edge of the superior anterior portion of the scapula.

The provision of a contact element which fits into or against such a feature such as the posterior notch or the coracoid process ensures a stable fixation of the surgical fixture onto the socket. For instance, in particular embodiments, the fixture is provided with a contact element fitting onto and/or into the posterior notch. The device can be designed such that, in order to place the contact element within the notch, the surgical fixture must be placed on the acetabulum in a first position whereby the contact element is placed next to the posterior notch. The desired final position of the surgical fixture onto the acetabulum is then obtained via a (slight) rotational movement of the fixture, which allows the contact element to move over and optionally into the notch until it reaches the rim of the notch. In particular embodiments, the contact between the contact element and the rim of the posterior notch of the transverse ligament ensures a rotational stop when the fixture is in the correct position. Thus, in particular embodiments, the fixtures, and more particularly a contact element thereof, may comprise a rotational undercut feature for registering on an anatomical feature around the socket, such as the posterior notch of the transverse ligament, or on top of the coracoid process.

Accordingly, in particular embodiments, the surgical fixtures are designed such that they comprise at least one contact element which interacts with an anatomical feature in the socket, on the socket rim or on the bone (with or without cartilage or other soft tissue) surrounding the socket. In comparative examples where the socket is a glenoid cavity, the bone surrounding the socket includes the glenoidal rim and the periglenoidal region (e.g. infraglenoidal tuberculum, supraglenoidal tuberculum and collum scapulae, etc.) and might include the acromion and coracoid process. Where the socket is an acetabulum, the bone surrounding the socket includes the periacetabular region (e.g. the limbus acetabuli, sulcus supra-acetabularis, superior ramus, etc.). In particular embodiments, the contact element is to be positioned on or within the posterior notch or the coracoid process. Specifically, one contact element is provided on the surgical fixture such that, when correctly positioned on the bone (i.e. in its final position), it fits within or onto the posterior notch or to the location of the coracoid process. More particularly, it is envisaged that, such a contact element grabs or locks onto the posterior notch or the coracoid process. This ensures a strong rotational stop when the fixture is in the correct position and therefore an accurate positioning of the fixture on the socket. Thus, in particular embodiments the shape of one contact element and its position on the fixture is such that, when positioned on the bone, it grabs onto or around the anatomical features such as the posterior notch or the coracoid process. Thus in particular embodiments such a contact element may comprise an undercut or may even be hook- shaped, such that it can grab on or around the anatomical feature. Thus, in particular embodiments one contact element comprises an undercut, or comprises a hook-shaped feature. In particular embodiments this undercut specifically mates with the acetabular notch.

In particular embodiments as described above, the one or more contact elements are designed such that correct positioning can be ensured by rotation of the contact elements over the rim of the socket. Thus, after placement of the contact elements on the bone, a slight rotation of the fixture will ensure that the contact elements fit into the planned position. Through the provision of an undercut in only one direction, the fixture can be rotated into position despite the fact that the contact elements are rigidly fixed to each other.

In further particular embodiments, it is envisaged that the contact element which interacts with a specific anatomical feature on the rim of the socket or the surrounding bone may further also provide a locking feature. Indeed, the shape of this contact element may be adapted such that, when positioned on the bone, it snaps onto and thus in fact locks onto this feature.

The surgical fixtures which may be manufactured according to the present invention allow correct positioning of an alignment element by way of the positioning element. For this purpose, the positioning element of the fixtures comprises an opening or hole which allows the insertion of an alignment element which is guided by the positioning element into the bone surrounding the socket, such as the pelvic bone or scapula. The alignment element may be a wire, pin, screw or drill, particularly a metal wire, pin, screw or drill. In particular embodiments, the alignment element is a wire or a pin, particularly a Kirschner wire (K- wire) or a Hoffmann pin.

In particular embodiments, the positioning element ensures placement of the alignment feature outside the socket. Indeed, in order to allow the guiding of the positioning of an implant for a socket of a ball-joint, the alignment element is typically positioned on the bone surrounding the socket, for example in the periacetabular area of the acetabulum (e.g. the limbus acetabuli, sulcus supra-acetabularis, superior ramus, etc.) or periglenoidal area of the glenoid cavity (e.g. infraglenoidal tuberculum, supraglenoidal tuberculum and collum scapulae, etc.) of the patient, in a direction parallel to the installation direction of the implant. For instance, for the positioning of an acetabular cup implant, the alignment element is typically positioned on the pelvic bone in a direction parallel to the installation direction of the acetabular implant. However, in particular embodiments, the positioning element ensures placement of the alignment feature inside (typically centrally in) the socket.

The optimal orientation of the opening of the positioning element can be obtained by determining the orientation of the positioning element according to pre-operational planning. Thus, in preferred embodiments, the direction and/ or the position of at least one hole of the surgical fixtures which allows the insertion of an alignment element is in accordance with pre-operational planning. More particularly, this implies that the relative position and/or orientation of least one hole relative to the contact element(s) of the surgical fixtures is in accordance with pre-operational planning. This allows the use of standard alignment elements such as K-wires. The positioning and/or orientation of the hole can be obtained via a certain location and/or orientation of the positioning element relative to the rest of the surgical fixture, via a certain location and/or position of the hole in the positioning element, or via a combination of the two. Additionally or alternatively, the shape of the alignment element itself can be provided such that, when inserted into the positioning element, it ensures the correct orientation to guide the implant.

Although the correct positioning of the surgical fixtures on the bone allows the insertion of an alignment element into the bone as soon as the correct positioning of the surgical fixture is obtained, the insertion of the alignment elements is significantly facilitated if the surgical fixtures are (temporarily) fixed to the bone and/or locked into the correct position. Thus, in particular embodiments, the surgical fixtures may comprise fixation features such as holes, which allow for fixation of the surgical fixtures onto the bone, for example using screws, wires or pins. Thus, in particular embodiments, the surgical fixtures comprise at least one hole in addition to the hole which is meant for insertion of the alignment element. In particular embodiments, the surgical fixtures comprise at least two holes. In certain embodiments, the hole(s) used as fixation features and the hole(s) used for insertion of the alignment element are located on the positioning element. In certain embodiments, the hole(s) used as fixation features and the hole(s) used for insertion of the alignment element are cylindrical. As both types of (cylindrical) holes have a different function, they may have a different diameter. The different diameter of the holes also avoids the surgeon from inserting the alignment element into a fixation feature, especially when the fixation features have a smaller diameter than the hole(s) for inserting an alignment element.

In particular embodiments of the fixtures, the positioning element and the one or more contact element(s) for positioning on the rim of the socket may be separate units which are detachable. However, when attached, the relative position between the contact element(s), the positioning element and the rest of the fixture is fixed.

In particular embodiments, the positioning element is located onto or integrated in a contact element, i.e. the structure comprising the positioning element has a surface which contacts with the bone. In particular embodiments however, the contact element is different from the one or more contact elements for placement on the rim of the socket. More particularly, it does not fit onto the rim of the socket but contacts a region outside the socket. A positioning element and such a contact element may form a single unit, thus the positioning element may also function as a contact element in that it helps to ensure accurate positioning on the bone.

In particular embodiments, the positioning element is integrated into or extends from a contact element on the rim of the socket. Where a positioning element is located onto a contact element on the rim of the socket, the hole(s) in the positioning element continue(s) through the contact element. In this way, the contact element does not block the insertion of fixation elements and/or alignment elements into the bone. Thus, in further embodiments, also the contact element(s) onto which a positioning element is located, contain at least one hole.

In some cases, the positioning of a second, or even a third alignment element allows an even more precise positioning of a socket implant. Thus, in particular embodiments, the surgical fixtures comprise more than one positioning element, of which at least one is detachable. In further embodiments, all positioning elements are detachable.

Insertion of the alignment element and/or fixation elements into the bone may be facilitated by first drilling a hole in the bone. The alignment element and/or fixation element(s) are then inserted into the hole drilled in the bone. Thus, in particular embodiments, at least one of the openings or holes on the positioning element (and the contact element(s) is part of a drill guide, which is located on the positioning element(s) or the contact element(s). In particular embodiments, the surgical fixture may be provided with a drill guide which can be positioned onto the hole.

In a particular embodiment, the surgical fixture is a disk (one contact element) comprising, on the side and/or edges for placement on the bone (with or without cartilage or other soft tissue), one or more contact points and/or contact surface(s) which match (i.e. specifically mate with) areas of (or around) the socket and/or the socket rim as described hereabove; and, on the opposing side of the contact surfaces, a positioning element. Advantageously, a less bulky fixture is obtained by omitting certain sectors from the disk, thus creating a surgical fixture containing two or more contact elements with one or more contact points and/or contact surfaces as described above. In particular embodiments, the contact elements may be interconnected by longitudinal structures from a central structure.

Thus, in certain embodiments, the surgical fixtures comprise a connecting structure, wherein one or more contact elements extend from the connecting structure. In further embodiments, two or more contact elements extend from the connecting structure. Also the positioning element(s) may extend from the connecting structure. In certain embodiments, the connecting structure is a central element, particularly a central axis. In particular embodiments, the contact element(s) and/or the positioning element(s) are separate units which are connectable to the connecting structure, central element or axis. In alternative embodiments, the surgical fixtures are manufactured as a single piece.

In particular embodiments, the fixture may contain extensions, positioned either on the contact elements and/or a central structure connecting the contact elements. The extensions increase grip on the fixture and visibility during the positioning of the fixture on the bone. Such extensions can vary in structure such as, but not limited to rod-like structures, hooks, etc. Typically, the extensions will extend from the fixture structure in a direction which is transversal to the contact surface of the contact elements.

In particular embodiments, the fixtures contain guiding features for guiding surgical instruments. As indicated above, such guiding features may include a drill bore for guiding a drill to make a hole in the bone for the alignment element. However, additional or alternative guiding elements may also be envisaged, including on one or more of the contact elements and/or a connecting structure (where applicable). Such guiding features include holes, slits grooves etc. It can further be envisaged that attachment features are provided on one or more parts of the fixture, for fixing additional elements, such as guiding features in a reversible way.

Once the alignment element is inserted into the bone via the positioning element(s), the surgical fixture must be removed from the socket in order to proceed with the surgery, e.g. to insert the socket implant. However, the inserted alignment element (and/or fixation elements) may limit the mobility of the surgical fixture, particularly when in the final position of the fixture one of the contact elements contacts or grabs an anatomical feature such as the posterior notch or the coracoid process. To overcome this problem, in particular embodiments of the surgical fixtures, at least one positioning element on the surgical fixture is detachable. Additionally, it is further envisaged in particular embodiments that one or more contact elements may be detachable.

In order to ensure a positioning element which is detachable from the contact elements or detachable contact elements, embodiments are envisaged wherein the connection between the positioning or contact element and the rest of the fixture is adapted or weakened to make the positioning or contact element detachable, while still maintaining the required rigidity to assure correct positioning. Thus, in particular embodiments, the connection between at least one positioning element or contact element and the rest of the fixture is adapted and/or weakened such that it can be detached from the guide with surgical cutting instruments. In certain embodiments, this connection is located on an extension of the connecting structure as described hereinabove.

Alternatively, the one or more positioning elements and/or contact elements may be removably connected to the rest of the fixture via a specific mechanism such as a dovetail coupling, interlocking features, a pinned system, a snap-fit system, or a combination thereof. This avoids the use of surgical cutting instruments. Moreover, if the connection between the positioning element(s) and/or the contact element(s) and the rest of the fixture is a standard connecting feature, the positioning element(s) can be reused on another surgical fixture. In certain embodiments, this connection is located on an extension of the connecting structure as described hereabove. In more particular embodiments, the positioning feature (which may itself comprise a contact surface so as to form a contact element) is connected to the remainder of the fixture comprising the one or more contact elements fitting on the rim of the socket by a standard connecting feature such as a dovetail coupling.

In particular embodiments, the surgical fixtures further comprise one or more extensions on the fixture which do not contain a contact element or positioning element. Such extensions as such do not contribute to the fit of the fixture on the bone but may allow an enhanced grip of the surgeon on the surgical fixtures, and may for example facilitate the rotational movement of the surgical fixture during its positioning, or facilitate the removal of the fixture after the positioning of the alignment element. For further facilitation of the rotational movement, such extensions may comprise a screw drive, i.e. a feature that allows the extension (and thus fixture) to be turned with a screw driver, hex key, or the like. Alternatively, the screw drive is not positioned on an extension, but elsewhere on the fixture.

The present invention provides methods for the manufacture of the surgical fixtures described herein.

The surgical fixtures comprise one or more patient-specific contact points and/or surfaces. Also the relative position and/or orientation of the positioning element(s) and/or the contact elements may be patient- specific. The generation of patient-specific surgical fixtures is done based on pre-operative images of the bone area (with or without cartilage or other soft tissue) surrounding the socket of the ball joint under consideration (e.g. the pelvic bone or the scapula), and planning of the surgery. More particularly, the generation of patient- specific surgical fixtures is done based on pre-operative images of the socket (e.g. the acetabulum or the glenoid cavity), and planning of the surgery. Accordingly, methods for producing the surgical fixtures according to the present invention typically comprise the steps of:
i. Obtaining volume information of a socket, e.g. the acetabulum or glenoid cavity, from a patient.
ii. Obtaining the installation direction of the socket implant for the patient.
iii. Identifying and selecting parts of the bone (with or without cartilage or other soft tissue) surrounding the implant zone which are suitable for inserting an alignment element.
iv. Identifying and selecting parts of the bone in or surrounding the implant zone which are suitable for use as a base for the contact points and/or contact surface(s) of the surgical fixture.
v. Designing and producing a surgical fixture based on the information obtained in steps i, ii and iii.

The step of obtaining volume information of the socket typically comprises obtaining digital patient-specific image information which can be done by any suitable means known in the art, such as for example a computer tomography (CT) scanner, a magnetic resonance imaging (MRI) scanner, an ultrasound scanner, or a combination of Roentgenograms. A summary of medical imaging has been described in "Fundamentals of Medical imaging", by P. Suetens, Cambridge University Press, 2002.

In a particular embodiment, Additive Manufacturing (AM) techniques are used for manufacturing the surgical fixtures, or parts thereof. AM techniques are particularly useful to manufacture patient-specific contact surfaces, or to produce the surgical fixtures in one piece. As an example, the manufacturing of medical- image-based patient-specific surgical instruments via AM is described in US Pat. No. 5.768.134 (Swaelens et al).

AM can be defined as a group of techniques used to fabricate a tangible model of an object typically using three-dimensional (3-D) computer aided design (CAD) data of the object. Currently, a multitude of Additive Manufacturing techniques is available, including stereolithography, Selective Laser Sintering, Fused Deposition Modeling, foil-based techniques, etc.

Selective laser sintering uses a high power laser or another focused heat source to sinter or weld small particles of plastic, metal, or ceramic powders into a mass representing the 3-dimensional object to be formed.

Fused deposition modeling and related techniques make use of a temporary transition from a solid material to a liquid state, usually due to heating. The material is driven through an extrusion nozzle in a controlled way and deposited in the required place as described among others in U.S. Pat. No. 5.141.680.

Foil-based techniques fix coats to one another by means of gluing or photo polymerization or other techniques and cut the object from these coats or polymerize the object. Such a technique is described in U.S. Pat. No. 5.192.539.
Typically AM techniques start from a digital representation of the 3-D object to be formed. Generally, the digital representation is sliced into a series of cross-sectional layers which can be overlaid to form the object as a whole. The AM apparatus uses this data for building the object on a layer-by-layer basis. The cross-sectional data representing the layer data of the 3-D object may be generated using a computer system and computer aided design and manufacturing (CAD/CAM) software.

The surgical fixtures may be manufactured in different materials. Typically, only materials that are biocompatible (e.g. USP class VI compatible) with the animal or human body are taken into account. Preferably the surgical fixture is formed from a heat-tolerable material allowing it to tolerate high-temperature sterilization. In the case selective laser sintering is used as an AM technique, the surgical template may be fabricated from a polyamide such as PA 2200 as supplied by EOS, Munich, Germany or any other material known by those skilled in the art may also be used.

Example methods for using the surgical fixtures described herein, for example for guiding an implant in a socket of a ball joint such as an acetabulum or glenoid cavity, comprise the steps of:
1) Positioning a surgical fixture as described herein onto the socket, e.g. acetabulum or glenoid cavity. This step may involve a (slight) rotational movement of the surgical fixture. The rotational movement facilitates obtaining the desired (pre-operationally planned) orientation of the fixture onto the socket;
2) Optionally fixing the surgical fixture onto the socket. This step is only possible if the surgical fixture comprises holes for inserting one or more fixation elements;
3) Using one of the holes provided by the positioning element of the surgical fixture to insert a pin, wire, drill or screw into the bone surrounding the socket;
4) Removing said surgical fixture from the socket, and
5) Using the pin, wire, drill or screw of step 3) to obtain the correct implant direction; this is the direction according to the pre-operational planning.

In particular examples, step 1 involves a rotational movement of the fixture to obtain the desired orientation onto the socket in such a way that the contact element which interacts with an anatomical feature on the rim of the socket is fitted into place. In particular examples, the rotational movement of the contact elements will allow the undercut or hook of the contact element to be tightly fixed onto an anatomical feature. More particularly for fixtures on the acetabulum, the placement of the fixture is envisaged to involve the fixing of the rotational undercut of a contact element of the device onto the posterior notch of the transverse ligament.

In particular examples, step 4 will comprise detaching said positioning element from the rest of the fixture and from the socket, so as to allow thereafter, a reversal of the rotational movement of step 1 to remove the rest of the fixture from the socket. In particular examples, this is ensured by releasing the coupling feature of the positioning element (e.g. dovetail). In alternative particular examples, this is ensured by breaking the connection between the positioning element and the rest of the fixture.

It is noted that the alignment element may be used as a visual alignment element or a physical alignment element. In particular embodiments, the alignment element is a pin or wire and is used as a physical alignment element to guide an implant or implant guide onto the bone in the correct position. Typically the implant or implant guide will comprise a hole or slit which is positioned such that, when the hole or slit of the implant or implant guide is mated with the alignment feature, it will guide the implant and/or implant guide directly in the desired position on the socket of the ball joint.

Surgical fixtures which may be manufactured using the methods of the invention will now be illustrated by the following, non-limiting illustrations of particular embodiments.

### EXAMPLES

### Example 1: surgical fixture with multiple contact elements.

The surgical fixtures may comprise more than one contact element. Figure 3 A shows such a surgical fixture (1) according to a particular embodiment, positioned onto the acetabulum of a pelvic bone (17), more particularly the pelvic bone of Figure 2B. Figures 3B and C show two views of the same fixture, not positioned on a bone.
The fixture comprises a positioning element (3) and four contact elements (2, 2'), each comprising a patient-specific contact surface (4). One of the contact surfaces (4') is designed to register onto the posterior notch of the transverse ligament (8), thereby providing a rotational undercut and a rotational lock. This facilitates the correct positioning of the fixture onto the acetabulum.
The positioning element forms a single unit with one of the contact elements (2'), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder. Each of the contact elements (2, 2') extends from a connecting structure (12). The connection (11) between the positioning element (3) and the connecting structure (12) is adapted, such that the positioning element can be easily detached from the surgical fixture with surgical cutting instruments. The surgical fixture (1) further comprises an additional extension (10), which allows for an enhanced grip on the fixture. The extension (10) is further provided with an identification code, for example a patient identifier.
Figure 3D shows a similar fixture as shown in Figures 3A-C, wherein the extension (10) is not provided with an identification code.

### Example 2: surgical fixture with one contact element.

The surgical fixtures may comprise only one contact element. Figure 4A shows such a surgical fixture (1) according to a particular embodiment. The fixture comprises a positioning element (3) and one contact element (2). The contact element comprises a patient-specific contact surface (4). The positioning element is attached to the contact element (2), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder. The connection between the positioning element (3) and the contact element (2) is adapted (not shown), such that the positioning element can be easily detached from the surgical fixture with surgical cutting instruments. The surgical fixture (1) further comprises two extensions (10), which are located on the contact element (2) and facilitate the rotational movement of the fixture during positioning. Figure 4 B shows the same fixture (1), positioned on an acetabulum of a pelvic bone (17). The contact surface on the contact element of the fixture surrounds the acetabulum over an angle of more than 180°.

### Example 3: surgical fixture providing rotational lock.

Figure 5A shows a surgical fixture (1) according to a particular embodiment, positioned onto the acetabulum of a pelvic bone (17), more particularly the pelvic bone of Figure 2B. Figures 5 Band C show two views of the same fixture, not positioned on a bone. The fixture comprises a positioning element (3) and four contact elements (2, 2'), each comprising a patient-specific contact surface (4, 4'). Each of the contact elements (2, 2') extends from a connecting structure (12). One of the contact surfaces (4') is designed to register onto the posterior notch of the transverse ligament (8). More particularly, the contact surface (4') is designed to partially surround the posterior notch (8), thereby providing a rotational undercut and a strong rotational lock. This significantly facilitates the correct and stable positioning of the fixture onto the acetabulum.
In the illustrated embodiment, the positioning element forms a single unit with one of the contact elements (2'), and comprises a hole (5) for insertion of an alignment element and holes (6) for insertion of fixation elements. The hole (5) for insertion of an alignment element may also be used for drilling a hole in the pelvic bone. Thus, the positioning element is also a drill guide (9), more particularly a drilling cylinder.
In this embodiment, the connection (11) between the positioning element (3) and the connecting structure (12) is adapted, such that the positioning element can be easily detached from the surgical fixture with surgical cutting instruments. The contact element (2') which forms a single unit with the positioning element (3) has a contact surface (4) which fits a part of the acetabular rim (7), but does not provide a rotational lock, as this would potentially impede the removal of the detached positioning element (3) from the acetabulum.
The surgical fixture (1) further comprises an additional extension (10), which allows for an enhanced grip on the fixture.

## Claims

1. A method of manufacturing a patient-specific surgical fixture (1) for positioning an alignment element on an acetabular socket of a pelvic bone, comprising the steps of:
a) obtaining volume information of the acetabular socket (13) of the pelvic bone (17) from a patient,
b) obtaining an installation direction of an acetabular socket implant for said patient,
c) identifying and selecting parts of a bone surrounding an implant zone which are suitable for inserting an alignment element,
d) identifying and selecting parts of a bone in or surrounding said implant zone which are suitable for use as a base for a contact surface or surfaces of the patient-specific surgical fixture (1), and
e) designing and producing the patient-specific surgical fixture (1) based on the information obtained in steps a, b, c and d, wherein the patient-specific surgical fixture (1) comprises:
a one-piece structure comprising one or more patient-specific contact elements (2, 2') which fit onto one or more areas on the acetabular socket (13), onto one or more areas around the acetabular socket (13) and/or onto a rim (7) of the acetabular socket (13),
wherein a surface of at least one of said one or more patient-specific contact elements (2, 2') is configured to correspond to a surface of a posterior notch of a transverse ligament (8) of the acetabular socket (13) such that, with the surface of the at least one of said one or more patient-specific contact elements (2, 2') being fitted into and/or onto the posterior notch (8) so as to engage with the surface of said posterior notch (8), the surface of the posterior notch (8) stops a rotation of the patient-specific surgical fixture (1); and
a positioning element (3) rigidly attached to said one-piece structure, provided with one or more holes (5) which allow the insertion of said alignment element.

2. The method according to claim 1, wherein the one or more holes (5) allow the insertion of said alignment element outside of said acetabular socket (13).

3. The method according to claims 1 or 2, wherein said positioning element (3) is detachable from the rest of said patient-specific surgical fixture (1).

4. The method according to any one of claims 1 to 3, wherein the one or more patient-specific contact elements (2, 2') comprises at least two patient-specific contact elements (2, 2'), which fit onto areas on the acetabular socket (13), onto one or more areas around said acetabular socket (13) and/or onto the rim (7) of said acetabular socket (13) in at least three contact points (4, 4'), wherein an angle between a line drawn between one contact point and a center of a circle or ellipse best fitting the rim (7) of said acetabular socket (13) and a line drawn between an adjacent contact point and said center is never greater than 180°.

5. The method according to any one of claims 1 to 4, wherein the one or more patient-specific contact elements (2, 2') fit onto areas on the rim (7) of said acetabular socket (13) in at least three contact points (4, 4') and are irreversibly fixed to each other.

6. The method according to any one of claims 1 to 5, wherein the one or more patient-specific contact elements (2, 2') comprises at least two patient-specific contact elements (2, 2') and wherein said positioning element (3) corresponds to one of said at least two patient-specific contact elements (2, 2').

7. The method according to any one of claims 1 to 6, wherein a connection (11) between said positioning element (3) and the rest of said patient-specific surgical fixture (1) is adapted or weakened, such that the positioning element (3) can be detached from the rest of said patient-specific surgical fixture (1) by breaking said connection (11) with surgical cutting instruments.

8. The method according to any one of claims 1 to 7, wherein a connection between said positioning element (3) and the rest of said patient-specific surgical fixture (1) comprises an element selected from a dovetail coupling, interlocking features, a pinned system and a snap-fit mechanism.

9. The method according to any one of claims 1 to 8, wherein the producing the patient-specific surgical fixture (1) comprises producing the patient-specific surgical fixture (1) to further comprise a connecting structure (12) and wherein said positioning element (3) and/or one or more of said one or more patient-specific contact elements (2, 2') extend from said connecting structure (12).

10. The method according to any one of claims 1 to 9, wherein a position and/or a direction of at least one of said one or more holes (5) is in accordance with pre-operational planning.

11. The method according to any one of claims 1 to 10, wherein at least one of said one or more holes (5) is part of a drill guide (9).

12. The method according to any one of claims 1 to 11, wherein said positioning element (3) comprises a first hole (5) and a second hole (6) with a different diameter than the first hole (5), wherein said first hole (5) allows the insertion of said alignment element, and wherein said second hole (6) allows the insertion of a fixation element.

13. The method according to any one of claims 1 to 12, wherein said alignment element is selected from the group comprising a pin, a wire, a screw and a drill.

14. The method according to any one of claims 1 to 13, wherein the producing said patient-specific surgical fixture (1) comprises producing said patient-specific surgical fixture (1) as one piece via additive manufacturing.

## Patentansprüche

1. Herstellungsverfahren einer patientenspezifischen chirurgischen Befestigung (1) zur Positionierung eines Ausrichtungselements an einer Acetabulumpfanne eines Beckenknochens, wobei das Verfahren die Schritte aufweist:
a) Erhalten von Volumeninformationen der Acetabulumpfanne (13) des Beckenknochens (17) von einem Patienten,
b) Erhalten einer Installationsrichtung eines Acetabulumpfannen-Implantats für den Patienten,
c) Identifizieren und Auswählen von Teilen eines eine Implantatzone umgebenden Knochens, die zum Einführen eines Ausrichtungselements geeignet sind,
d) Identifizieren und Auswählen von Teilen eines in der Implantatzone gelegenen oder die Implantatzone umgebenden Knochens, die zur Nutzung als eine Basis für eine Kontaktfläche oder für Oberflächen der patientenspezifischen chirurgischen Befestigung (1) geeignet sind, und
e) Entwerfen und Erzeugen der patientenspezifischen chirurgischen Befestigung (1) basierend auf den in den Schritten a, b, c und d erhaltenen Informationen, wobei die patientenspezifische chirurgische Befestigung (1) aufweist:
eine einteilige Struktur, die ein oder mehrere patientenspezifische Kontaktelemente (2, 2') aufweist, die auf einen oder mehrere Bereiche an der Acetabulumpfanne (13), auf einen oder mehrere Bereiche um die Acetabulumpfanne (13) und/oder auf einen Rand (7) der Acetabulumpfanne (13) passen,
wobei eine Oberfläche von wenigstens einem des einen oder der mehreren patientenspezifischen Kontaktelemente (2, 2') dergestalt ist, einer Oberfläche einer posterioren Aussparung eines transversalen Bandes (8) der Acetabulumpfanne (13) zu entsprechen, so dass, mit der Oberfläche von dem wenigstens einen des einen oder der mehreren patientenspezifischen Kontaktelemente (2, 2') in und/oder auf die posteriore Aussparung (8) passend, um mit der Oberfläche der posterioren Aussparung einzurasten, die Oberfläche der posterioren Aussparung (8) eine Rotation der patientenspezifischen chirurgischen Befestigung (1) stoppt; und
ein Positionierungselement (3) starr befestigt an der einteiligen Struktur und versehen mit einem oder mehreren Löchern (5), die die Einführung des Ausrichtungselements ermöglichen.

2. Verfahren gemäß Anspruch 1, wobei das eine und die mehreren Löcher (5) die Einführung des Ausrichtungselements außerhalb der Acetabulumpfanne (13) ermöglichen.

3. Verfahren gemäß Anspruch 1 oder 2, wobei das Positionierungselement (3) von dem Rest der patientenspezifischen chirurgischen Befestigung (1) abtrennbar ist.

4. Verfahren gemäß einem der Ansprüche 1 bis 3, wobei das eine oder die mehreren patientenspezifischen Kontaktelemente (2,2') wenigstens zwei patientenspezifische Kontaktelemente (2, 2') aufweisen, die auf Bereiche an der Acetabulumpfanne (13), auf einen oder mehrere Bereiche um die Acetabulumpfanne (13) und/oder auf den Rand (7) der Acetabulumpfanne (13) an wenigstens drei Kontaktpunkten (4,4') passen, wobei ein Winkel zwischen einer zwischen einem Kontaktpunkt und einem Zentrum eines Kreises oder einer Ellipse, der oder die dem Rand (7) der Acetabulumpfanne (13) am besten entspricht, gezeichneten Linie und einer zwischen einem angrenzenden Kontaktpunkt und dem Zentrum gezeichneten Linie nie größer als 180° ist.

5. Verfahren gemäß einem der Ansprüche 1 bis 4, wobei das eine oder die mehreren patientenspezifischen Kontaktelemente (2, 2') auf Bereiche an dem Rand (7) der Acetabulumpfanne (13) an wenigstens drei Kontaktpunkten (4, 4') passen und unabänderlich zueinander fixiert sind.

6. Verfahren gemäß einem der Ansprüche 1 bis 5, wobei das eine oder die mehreren patientenspezifischen Kontaktelemente (2, 2') wenigstens zwei patientenspezifische Kontaktelemente (2, 2') aufweisen und wobei das Positionierungselement (3) einem der wenigstens zwei patientenspezifischen Kontaktelemente (2, 2') entspricht.

7. Verfahren gemäß einem der Ansprüche 1 bis 6, wobei eine Verbindung (11) zwischen dem Positionierungselement (3) und dem Rest der patientenspezifischen chirurgischen Befestigung (1) angepasst oder geschwächt ist, so dass das Positionierungselement (3) von dem Rest der patientenspezifischen chirurgischen Befestigung (1) durch Brechen der Verbindung (11) mit chirurgischen scharfen Instrumenten abgetrennt werden kann.

8. Verfahren gemäß einem der Ansprüche 1 bis 7, wobei eine Verbindung zwischen dem Positionierungselement (3) und dem Rest der patientenspezifischen chirurgischen Befestigung (1) ein Element ausgewählt aus einer Schwalbenschwanz-Kupplung, ineinandergreifenden Merkmalen, einer gesteckten Vorrichtung und einem Schnappmechanismus aufweist.

9. Verfahren gemäß einem der Ansprüche 1 bis 8, wobei das Erzeugen der patientenspezifischen chirurgischen Befestigung (1) das Erzeugen der patientenspezifischen chirurgischen Befestigung (1) aufweist, um weiter eine Verbindungstruktur (12) aufzuweisen, und wobei sich das Positionierungselement (3) und/oder eines oder mehrere des einen oder der mehreren patientenspezifischen Kontaktelemente (2, 2') von der Verbindungstruktur (12) erstreckt.

10. Verfahren gemäß einem der Ansprüche 1 bis 9, wobei eine Position und/oder eine Richtung von wenigstens einem des einen oder der mehreren Löcher (5) entsprechend präoperativer Planung ist.

11. Verfahren gemäß einem der Ansprüche 1 bis 10, wobei wenigstens eines des einen oder der mehreren Löcher (5) Teil einer Bohrführung (9) ist.

12. Verfahren gemäß einem der Ansprüche 1 bis 11, wobei das Positionierungselement (3) ein erstes Loch (5) und ein zweites Loch (6) mit einem anderen Durchmesser als das erste Loch (5) aufweist, wobei das erste Loch (5) die Einführung des Ausrichtungselements ermöglicht und wobei das zweite Loch (6) die Einführung eines Fixierungselements ermöglicht.

13. Verfahren gemäß einem der Ansprüche 1 bis 12, wobei das Ausrichtungselement aus der Gruppe ausgewählt ist, die einen Bolzen, einen Draht, eine Schraube und einen Bohrer aufweist.

14. Verfahren gemäß einem der Ansprüche 1 bis 13, wobei das Erzeugen der patientenspezifischen chirurgischen Befestigung (1) das Erzeugen der patientenspezifischen chirurgischen Befestigung (1) als ein Teil durch additive Herstellung aufweist.

## Revendications

1. Procédé pour fabriquer un dispositif de fixation chirurgical spécifique au patient (1) afin de positionner un élément d'alignement sur une cavité acétabulaire d'un os pelvien, comprenant les étapes suivantes qui consistent à :
a) obtenir l'information de volume de la cavité acétabulaire (13) de l'os pelvien (17) d'un patient,
b) obtenir une direction d'installation d'un implant de cavité acétabulaire pour ledit patient,
c) identifier et sélectionner des parties d'un os entourant une zone d'implant qui sont appropriées pour insérer un élément d'alignement,
d) identifier et sélectionner des parties d'un os dans ou entourant ladite zone d'implant qui sont appropriées pour être utilisées en tant que base pour une surface ou des surfaces de contact du dispositif de fixation chirurgical spécifique au patient (1), et
e) concevoir et produire le dispositif de fixation chirurgical spécifique au patient (1) sur la base de l'information obtenue aux étapes a, b, c et d, où le dispositif de fixation chirurgical spécifique au patient (1) comprend :
une structure d'un seul tenant comprenant un ou plusieurs éléments de contact spécifiques au patient (2, 2') qui se montent sur une ou plusieurs zones sur la cavité acétabulaire (13), sur une ou plusieurs zones autour de la cavité acétabulaire (13) et/ou sur un bord (7) de la cavité acétabulaire (13),
dans lequel une surface d'au moins l'un desdits un ou plusieurs éléments de contact spécifiques au patient (2, 2') est configurée pour correspondre à une surface d'une encoche postérieure d'un ligament transversal (8) de la cavité acétabulaire (13) de sorte que, avec la surface de l'au moins un desdits un ou plusieurs éléments de contact spécifiques au patient (2, 2') qui est montée dans et/ou sur l'encoche postérieure (8) afin de se mettre en prise avec la surface de ladite encoche postérieure (8), la surface de l'encoche postérieure (8) arrête une rotation du dispositif de fixation chirurgical spécifique au patient (1) ; et
un élément de positionnement (3) rigidement fixé à ladite structure d'un seul tenant, prévu avec un ou plusieurs trous (5) qui permettent l'insertion dudit élément d'alignement.

2. Procédé selon la revendication 1, dans lequel lesdits un ou plusieurs trous (5) permettent l'insertion dudit élément d'alignement à l'extérieur de ladite cavité acétabulaire (13).

3. Procédé selon la revendication 1 ou 2, dans lequel ledit élément de positionnement (3) est détachable du reste dudit dispositif de fixation chirurgical spécifique au patient (1).

4. Procédé selon l'une quelconque des revendications 1 à 3, dans lequel les un ou plusieurs éléments de contact spécifiques au patient (2, 2') comprennent au moins deux éléments de contact spécifiques au patient (2, 2'), qui se montent sur des zones sur la cavité acétabulaire (13), sur une ou plusieurs zones autour de ladite cavité acétabulaire (13), et/ou sur le bord (7) de ladite cavité acétabulaire (13) dans au moins trois points de contact (4, 4'), dans lequel un angle entre une ligne tracée entre un point de contact et un centre d'un cercle ou d'une ellipse mieux adapté au bord (7) de ladite cavité acétabulaire (13) et une ligne tracée entre un point de contact adjacent et ledit centre n'est jamais supérieur à 180°.

5. Procédé selon l'une quelconque des revendications 1 à 4, dans lequel les un ou plusieurs éléments de contact spécifiques au patient (2, 2') se montent sur des zones sur le bord (7) de ladite cavité acétabulaire (13) dans au moins trois points de contact (4, 4') et sont fixés de manière irréversible entre eux.

6. Procédé selon l'une quelconque des revendications 1 à 5, dans lequel les un ou plusieurs éléments de contact spécifiques au patient (2, 2') comprennent au moins deux éléments de contact spécifiques au patient (2, 2') et dans lequel ledit élément de positionnement (3) correspond à l'un desdits au moins deux éléments de contact spécifiques au patient (2, 2').

7. Procédé selon l'une quelconque des revendications 1 à 6, dans lequel un raccordement (11) entre ledit élément de positionnement (3) et le reste dudit dispositif de fixation chirurgical spécifique au patient (1) est adapté ou affaibli, de sorte que l'élément de positionnement (3) peut être détaché du reste dudit dispositif de fixation chirurgical spécifique au patient (1) en cassant ledit raccordement (11) avec des instruments de coupe chirurgicaux.

8. Procédé selon l'une quelconque des revendications 1 à 7, dans lequel un raccordement entre ledit élément de positionnement (3) et le reste dudit dispositif de fixation chirurgical spécifique au patient (1) comprend un élément sélectionné parmi un couplage à queue d'aronde, des caractéristiques de verrouillage, un système à broche et un mécanisme à encliquetage.

9. Procédé selon l'une quelconque des revendications 1 à 8, dans lequel l'étape pour produire le dispositif de fixation chirurgical spécifique au patient (1) comprend l'étape pour produire le dispositif de fixation chirurgical spécifique au patient (1) afin de comprendre en outre une structure de raccordement (12) et dans lequel ledit élément de positionnement (3) et/ou un ou plusieurs desdits un ou plusieurs éléments de contact spécifiques au patient (2, 2') s'étendent à partir de ladite structure de raccordement (12).

10. Procédé selon l'une quelconque des revendications 1 à 9, dans lequel une position et/ou une direction d'au moins l'un desdits un ou plusieurs trous (5) est/sont selon la planification préopératoire.

11. Procédé selon l'une quelconque des revendications 1 à 10, dans lequel au moins l'un desdits un ou plusieurs trous (5) fait partie d'un guide de perçage (9).

12. Procédé selon l'une quelconque des revendications 1 à 11, dans lequel ledit élément de positionnement (3) comprend un premier trou (5) et un second trou (6) avec un diamètre différent du premier trou (5), dans lequel ledit premier trou (5) permet l'insertion dudit élément d'alignement, et dans lequel ledit second trou (6) permet l'insertion d'un élément de fixation.

13. Procédé selon l'une quelconque des revendications 1 à 12, dans lequel ledit élément d'alignement est sélectionné dans le groupe comprenant une broche, un fil, une vis et un foret.

14. Procédé selon l'une quelconque des revendications 1 à 13, dans lequel l'étape pour produire ledit dispositif de fixation chirurgical spécifique au patient (1) comprend l'étape pour produire ledit dispositif chirurgical spécifique au patient (1) d'un seul tenant via une fabrication additive.
